# EUROPÄISCHE PATENTANMELDUNG

(11) **EP 3 708 129 A1**
(43) Veröffentlichungstag der Anmeldung: **16.09.2020**
(21) Anmeldenummer: 19163172.0
(22) Anmeldetag: 15.03.2019
(51) Int. Cl.: A61F 2/915

(54) **IMPLANTAT**

(71) Anmelder: BIOTRONIK AG, 8180 Bülach (CH)
(72) Erfinder: Schoof, André, 18209 Bad Doberan (DE); Fricke, Dirk, 18055 Rostock (DE)
(74) Vertreter: Galander, Marcus

(57) **Zusammenfassung**

Die Erfindung betrifft ein Implantat mit einem durchbrochenen, hohlzylinderförmigen Grundkörper (10), der sich aus einer Vielzahl von durchbrochenen, hohlzylinderförmigen, in Längsrichtung (L) aneinandergereihten Segmenten (11, 13) zusammensetzt, die mit einander verbunden sind. Die Segmente umfassen jeweils ein endständiges Segment (11) an jedem in Längsrichtung angeordneten Ende des Grundkörpers (10) und mindestens ein innenliegendes Segment (13), das in Längsrichtung (L) zwischen den zwei endständigen Segmenten (11) angeordnet ist, wobei jedes Segment (11, 13) eine Vielzahl von Stegen (21) aufweist, die zusammen eine in Umfangsrichtung umlaufende mäanderförmige Struktur mit Maxima (25) und Minima (23) ausbilden. Der Grundkörper (10) nimmt einen komprimierten Zustand und einen expandierten Zustand ein. Die mäanderförmige Struktur mindestens eines endständigen Segments (11) weist eine geringere Anzahl von Maxima (25) und Minima (23) auf als das mindestens eine innenliegende Segment (13) und der Innen- oder Außendurchmesser dieses mindestens einen endständigen Segments (11) ist im komprimierten Zustand oder im expandierten Zustand im Wesentlichen gleich dem Innen- oder Außendurchmesser des mindestens einen innenliegenden Segments (13) im gleichen Zustand.

## Beschreibung

Die vorliegende Erfindung betrifft ein Implantat mit einem durchbrochenen, hohlzylinderförmigen Grundkörper, der sich aus einer Vielzahl von durchbrochenen, hohlzylinderförmigen, in Längsrichtung aneinandergereihten Segmenten zusammensetzt, die miteinander verbunden sind, wobei jedes Segment eine Vielzahl von Stegen aufweist, die zusammen eine in Umfangsrichtung umlaufende mäanderförmige Struktur mit Minima und Maxima ausbilden. Das Implantat bzw. dessen Grundkörper kann einen komprimierten Zustand und einen expandierten Zustand einnehmen.

Medizinische Implantate (Prothesen), insbesondere intraluminale Endoprothesen, für unterschiedlichste Anwendungen sind in großer Vielfalt aus dem Stand der Technik bekannt.

Als Implantate werden häufig Stents eingesetzt, die beispielsweise zur Behandlung von Stenosen (Gefäßverengungen) dienen können. Sie weisen meist einen durchbrochenen hohlzylinderförmigen (rohrförmigen) Grundkörper auf, der an beiden Enden in Längsrichtung (d.h. in Richtung der Längsachse) offen ist. Ein derartiges Implantat wird häufig mittels eines Katheters in das zu behandelnde Gefäß eingesetzt und dient dazu, das Gefäß mittels seines Grundkörpers über einen längeren Zeitraum (Monate bis Jahre) zu stützen. Durch den Einsatz von Stents können verengte Bereiche in den Gefäßen erweitert werden.

Zudem bereits bekannt sind Implantate, insbesondere Stents, die teilweise oder vollständig aus einem biodegradierbaren Material bestehen. Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Aus der Druckschrift EP 1 974 700 A1 ist ein Implantat in Form eines Stents mit einem radial expandierbaren Grundkörper bekannt, wobei der Grundkörper eine Vielzahl von Tragsegmenten aufweist. Jedes Segment wird durch mäanderförmige Streben ausgebildet. Auch die Druckschrift EP 3 034 035 A1 beschreibt ein solches Implantat, das flexibel ist und gleichzeitig eine ausreichende radiale Steifigkeit aufweist, um das Gefäß, in das es eingesetzt ist, zu stützen.

Bekannten Implantate wie die oben erwähnten, deren Grundkörper aus einer Vielzahl von Segmenten zusammengesetzt ist, die sich in Längsrichtung aneinanderreihen, besitzen häufig in jedem Segment eine Vielzahl von Streben, die jeweils eine umlaufende, mäanderförmige Struktur ausbilden. Hierfür sind die Streben aneinander befestigt bzw. gehen an den jeweiligen bogenförmigen Maxima und Minima der Struktur ineinander über. Die mäanderförmige Struktur besteht aus mehreren einzelnen Minima und Maxima. Die mäanderförmige Struktur besitzt abwechselnd Minima und Maxima, wobei als Minima eines Segmentes diejenigen Bögen bezeichnet werden, welche näher an einem ersten Ende des Grundkörpers liegen während als Maxima die jeweiligen anderen Bögen bezeichnet werden, die näher an dem Ende des Grundkörpers liegen, welches dem ersten Ende entgegengesetzt ist.

Im Rahmen dieser Anmeldung wird unter einem Segment ein hohlzylinderförmiger Abschnitt des Implantats verstanden. Das Segment besteht aus einer Vielzahl von Streben und Bögen, die eine umlaufende, mäanderförmige Struktur bilden. Das Segment ist in Umfangsrichtung geschlossen, das heißt die Streben und Bögen eines Segmentes sind derart miteinander verbunden, dass sie einen in Umfangsrichtung geschlossenen Ring bilden. Bei einem Stent wird eine derartige Ausgestaltung auch häufig als Ringdesign bezeichnet.

Die bekannten Implantate nehmen einerseits den oben bereits erwähnten komprimierten Zustand ein, in welchem sie minimalinvasiv in den Körper des Behandelten, z.B. mittels eines Katheters, eingebracht werden können. Andererseits, am Ort der Behandlung, an dem das Implantat verbleiben soll, sind die Implantate in den expandierten Zustand überführt, beispielsweise mittels Ballondilatation. Im expandierten Zustand stützen die Implantate mittels ihres Grundkörpers das jeweilige Gefäß oder sind in dem jeweiligen Organ oder sonstigem Körperhohlraum verankert. Unter dem expandierten Zustand wird im Rahmen der Anmeldung der Zustand des Implantates verstanden, der sich nach Expansion beispielsweise mittels Ballondilation ergibt, wobei individuelle und lokale äußere Einflüsse durch den individuellen Implantationsort (beispielsweise lokal unterschiedliche Gefäßdurchmesser, Steifigkeiten oder Abweichungen vom kreisförmigen Querschnitt) nicht berücksichtigt werden. Der expandierte Zustand entspricht daher einem Zustand nach Expansion in Luft.

Im Rahmen der nachfolgenden Erläuterung werden mit dem Begriff "Gefäß" alle Gefäße, Organe oder sonstige Körperhohlräume, insbesondere Blutgefäße, des Behandelten zusammengefasst, in die ein gattungsgemäßes Implantat zur Behandlung eingesetzt werden kann.

Wenn ein Implantat mit dem oben beschriebenen Grundkörper in ein Gefäß eingesetzt ist, so strömt die Körperflüssigkeit dieses Gefäßes durch das Implantat. Im Übergang zwischen dem Gefäß ohne Implantat und Gefäß mit Implantat treten Strömungsänderungen auf, welche die Strömung der Körperflüssigkeit behindern und ggf. zur unerwünschten Ablagerung oder Agglomeration von Inhaltsstoffen dieser Körperflüssigkeit im Bereich dieses Übergangs führen können. Bei einem beweglichen Gefäß (z.B. einem Blutgefäß) verändert sich im Bereich des eingesetzten Implantats zudem die Steifigkeit des jeweiligen Gefäßes, da zu der Steifigkeit auch das an der Behandlungsstelle vorhandene Implantat beiträgt. Diese Veränderung der Steifigkeit soll nach Möglichkeit nicht sprunghaft erfolgen.

Die Aufgabe besteht somit darin, ein Implantat zu schaffen, das einen besseren Strömungsübergang und einen geringeren Steifigkeitssprung beim Übergang von dem Gefäß ohne Implantat zu dem Gefäß mit Implantat erzielt. Ferner soll das erfindungsgemäße Implantat einfacher und besser implantierbar sein als vergleichbare Implantate des Standes der Technik.

Die obige Aufgabe wird gelöst durch ein Implantat mit den Merkmalen des Anspruchs 1.

Insbesondere besitzt das erfindungsgemäße Implantat eine Vielzahl von durchbrochenen, hohlzylinderförmigen, in Längsrichtung aneinandergereihten Segmenten mit einer oben beschriebenen mäanderförmigen Struktur, wobei die Segmente jeweils ein endständiges Segment an jedem in Längsrichtung angeordneten Ende des Grundkörpers und mindestens ein innenliegendes Segment umfassen, das in Längsrichtung zwischen den zwei endständigen Segmenten angeordnet ist. Erfindungsgemäß weist die mäanderförmige Struktur mindestens eines endständigen Segments eine geringere Anzahl von Minima auf als das mindestens eine innenliegende Segment.

Vorzugsweise weist ein Segment genauso viele Minima wie Maxima auf. Dies gilt bevorzugt genauso für die endständigen Segmente wie für die innenliegenden Segmente.

Der Außendurchmesser dieses mindestens einen endständigen Segments ist vorzugsweise im expandierten Zustand im Wesentlichen gleich dem Außendurchmesser des mindestens einen innenliegenden Segments im gleichen Zustand. Mit anderen Worten, ergibt sich bei Expansion des Implantats ein hohlzylindrischer Grundkörper dessen Außendurchmesser sich zwischen innenliegenden Segmenten und Endsegmenten nicht unterscheidet. Unter im Wesentlichen gleicher Außendurchmesser wird im Rahmen dieser Anmeldung eine Durchmesserunterschied von weniger als 3%, bevorzugt weniger als 2% verstanden.

Durch die erfindungsgemäße Lösung wird ein Implantat geschaffen, dessen Grundkörper eine verbesserte Implantationsprozedur ermöglicht. Da die Struktur der Segmente mäanderförmig ist, bedeutet die Reduktion der Anzahl der Minima in dem jeweiligen endständigen Segment gleichzeitig auch eine entsprechende Reduktion der Anzahl der Maxima und der Stege. Dies bedeutet, dass das endständige Segment auf einen kleineren Durchmesser komprimiert werden kann als die innenliegenden Segmente. Bei der Implantation wird das Implantat mit einem endständigen Segment voran in das Gefäß eingeführt. Durch den geringeren komprimierten Durchmesser des endständigen Segmentes wird das Risiko minimiert, dass das endständige Segment absteht und beim Einführen gegen die Gefäßwand stößt und diese traumatisiert. Zudem wird durch die geringere Anzahl der Stege im Bereich des jeweiligen endständigen Segments ein besserer Strömungsübergang geschaffen.

In einem Ausführungsbeispiel der Erfindung weisen beide endständige Segmente eine geringere Anzahl von Minima auf als das mindestens eine innenliegende Segment.

In einem weiteren Ausführungsbeispiel ist die Anzahl der Minima des einen endständigen Segments oder der beiden endständigen Segmente um 1 oder 2 kleiner als die Anzahl der Minima der innenliegenden Segmente. Wenn die innenliegenden Segmente unterschiedliche Anzahlen von Minima aufweisen, dann soll in einem Ausführungsbeispiel die Anzahl der Minima des einen endständigen Segments oder der beiden endständigen Segmente um 1 oder 2 kleiner als die Anzahl der Minima des innenliegenden Segments mit der kleinsten Anzahl von Minima sein.

Beispielsweise weist das Implantat innenliegende Segmente auf, die jeweils 6 Minima in der mäanderförmigen Struktur ausbilden. In diesem Ausführungsbeispiel besitzen ein endständiges Segment oder beide endständigen Segmente beispielsweise 4 oder 5 Minima. Andere Anzahlen von Minima sind von der obigen Definition mit umfasst, wobei die Anzahl der Minima eine natürliche Zahl ist.

In einem Ausführungsbeispiel ist an der mäanderförmigen Struktur mindestens eines endständigen Segments mindestens eine Öse für die Anordnung eines Funktionselements bestehend aus einem röntgenopaken und/oder radioopaken Materials vorgesehen, wobei die innere Öffnung der Öse vorzugsweise eine elliptische Form aufweist. Es ist ebenfalls eine Öse mit einer runden Form der inneren Öffnung oder einer quadratischen Form oder einer rechteckigen Form oder einer anderen Form der inneren Öffnung denkbar. Die Anordnung eines in Bezug auf herkömmliche Implantate vergleichsweise großen röntgenopaken und/oder radioopaken Funktionselements ist bei dem erfindungsgemäßen Implantat möglich, da durch die reduzierte Minimaanzahl und damit reduzierte Steganzahl der erforderliche Platz hierfür geschaffen wurde, ohne die Komprimierung oder Expansion des Grundkörpers in diesem Bereich zu behindern. Die äußere Länge der Öse, d.h. die äußere Abmessung in Längsrichtung, ist hierbei kleiner oder gleich der Breite des jeweiligen endständigen Segments. Hierbei wird als Breite eines Segments (Segmentbreite) die äußere Länge des Segments in dem jeweiligen Zustand in Längsrichtung des Grundkörpers bezeichnet. In dieser Ausgestaltung kommen die Vorteile der Erfindung in Bezug auf einen kleinen Durchmesser im komprimierten Zustand besonders zur Geltung. Durch die reduzierte Anzahl der Minima wird mehr Platz für das Funktionselement geschaffen, so dass das Implantat mit Funktionselement insgesamt auf einen kleineren Durchmesser komprimiert werden kann. Das Funktionselement vergrößert nicht den Durchmesser im komprimierten Zustand. Dies führt zu einem kleinen Profil eines Kathetersystems zur Einführung eines derartigen Implantats und ermöglicht so eine einfachere und weniger traumatische Implantation.

Das Funktionselement kann beispielsweise aus einem oder mehreren röntgen- und/oder radioopaken Elementen oder Verbindungen aus der Gruppe umfassend Platin, Iridium, Gold, Wolfram, Molybdän, Niob, Tantal, Yttrium, Zirkonium, Ytterbium oder Legierungen aus diesen Metallen bestehen.

In einem Ausführungsbeispiel ist jeweils ein Funktionselement bestehend aus einem röntgenopaken und/oder radioopaken Material in der mindestens einen Öse angeordnet. Hierbei ist das Funktionselement, beispielsweise mittels einer Klebeverbindung, an der Öse befestigt.

In einem Ausführungsbeispiel kann eine weitere Verringerung des Steifigkeitssprungs zwischen Gefäß mit Implantat und Gefäß ohne Implantat dadurch erreicht werden, dass mindestens ein endständiges Segment eine Segmentbreite aufweist, die größer ist als die Segmentbreite des mindestens einen innenliegenden Segments. Durch die größere Segmentbreite des mindestens einen endständigen Segments weist diese eine weiter reduzierte radiale Steifigkeit auf. Die Segmentbreite des endständigen Segments kann beispielsweise zwischen 1 mm und 2 mm, bevorzugt zwischen 1,1 mm und 1,6 mm betragen. Die Segmentbreite des innenliegenden Segments kann beispielsweise in einem ähnlichen Bereich liegen. Dabei weist/weisen das/die innenliegende Segment(e) vorteilhafterweise eine zwischen 0,01 mm und 0,3 mm, bevorzugt zwischen 0,01 mm und 0,2 mm, besonders bevorzugt eine zwischen 0,07 mm bis 0,12 mm, geringere Stegbreite auf.

In einem weiteren Ausführungsbeispiel ist der Abstand eines endständigen Segments von dem benachbarten innenliegenden Segment größer als der Abstand zweier benachbarter innenliegender Segmente. Hierdurch wird die axiale Steifigkeit in dem Bereich des jeweiligen endständigen Segments verringert und so ein besserer Steifigkeitsübergang geschaffen. Hierbei wird als Abstand zwischen zwei Segmenten die minimale Länge des Leerraums in Längsrichtung zwischen der Linie, welche die Minima des einen Segments verbindet, und der Linie verstanden, welche die gegenüber liegenden Maxima des anderen Segments verbindet. Der Abstand des endständigen Segments von dem benachbarten innenliegenden Segment in Längsrichtung kann beispielsweise zwischen 0,10 mm und 0,13 mm, bevorzugt zwischen 0,11 mm und 0,12 mm, betragen. Der Abstand zweier benachbarter innenliegender Segmente in Längsrichtung kann beispielsweise zwischen 0,08 mm und 0,12 mm, bevorzugt zwischen 0,09 mm und 0,11 mm betragen.

In einem weiteren Ausführungsbeispiel weisen die Stege mindestens eines endständigen Segments eine geringere Breite und/oder eine stärkere Verrundung der Kanten auf als die Stege des mindestens einen innenliegenden Segments. Durch diese Ausbildung der Stege der Segmente wird einerseits ein noch besserer Strömungsübergang in Richtung des Inneren des Implantats geschaffen, da die Materialstärke im Bereich des jeweiligen endständigen Bereichs verringert wird. Andererseits wird die Radialsteifigkeit im Bereich des jeweiligen endständigen Segments weiter reduziert. Als Stegbreite wird die Breite des Stegs in einer Ebene gemessen, die entsteht, wenn der sich über einen Umfang eines Hohlzylinders erstreckende Grundkörper in eine Ebene abgerollt wird (Abrollebene). Hierbei erfolgt die Messung der Breite des jeweiligen Stegs in dieser Abrollebene senkrecht zu den jeweiligen Außenflächen des Stegs, die senkrecht zur Abrollebene liegen. Die geringere Breite der Stege und/oder stärkere Verrundung der Kanten in dem jeweiligen endständigen Segment kann beispielsweise durch eine entsprechende Elektropolitur in diesem Bereich erreicht werden. Die Stegbreite der Stege des endständigen Segments kann beispielsweise zwischen 0,135 mm und 0,155 mm, bevorzugt zwischen 0,140 mm und 0,150 mmm betragen. Die Stegbreite der Stege des innenliegenden Segments kann beispielsweise zwischen 0,145 mm und 0.160 mm, bevorzugt 0,150 mm und 0,155 mm, betragen. Unter einer stärkeren Verrundung der Kanten wird im Rahmen dieser Anmeldung eine abgerundete Kante mit größerem Radius des Kreisbogens der Verrundung verstanden.

In einem weiteren Ausführungsbeispiel der Erfindung weisen die Stege mindestens eines endständigen Segments eine geringere Dicke auf als die Stege des mindestens einen innenliegenden Segments. Analog zur Verringerung der Stegbreite hat eine Verringerung der Stegdicke einen besseren Strömungsübergang in Richtung des Implantatinneren sowie eine Verringerung der Radialsteifigkeit zur Folge, wodurch ein besserer Übergang zwischen dem Gefäß ohne Implantat und dem Gefäß mit Implantat erreicht wird. Unter der Stegdicke wird im Rahmen dieser Anmeldung die Wandstärke des Steges verstanden, die sich bei Betrachtung des Querschnittes eines Steges senkrecht zur Stegbreite ergibt. Eine geringere Stegdicke in dem jeweiligen endständigen Element lässt sich ebenfalls durch eine geeignete Elektropolitur erreichen. Die Stegdicke des endständigen Segments kann beispielsweise 5 µm bis 15 µm geringer ausfallen als die Stegdicke eines innenliegendes Segmentes.

Bevorzugt werden die beiden vorangehend geschilderten Ausführungsformen kombiniert. In dieser bevorzugten Kombination weisen die Stege mindestens eines endständigen Segments eine geringere Breite, eine geringere Dicke und/oder eine stärkere Verrundung der Kanten auf.

In einem weiteren Ausführungsbeispiel sind benachbarte Segmente mittels mindestens eines Verbindungsstegs miteinander verbunden, wobei der mindestens eine Verbindungssteg eine geringere Stegbreite und/oder Stegdicke aufweist als die Stege des mindestens einen innenliegenden Segments und/oder die Stege des mindestens einen endständigen Segments. Hierdurch erhält das Implantat in vorteilhafter Weise insgesamt eine gute axiale Flexibilität sowie eine gute Biegeflexibilität. Die Stegbreite der Verbindungsstege kann beispielsweise zwischen 0,07 mm und 0,10 mm, bevorzugt zwischen 0,08 mm und 0,09 mm, betragen und wird analog zur Breite der Stege der Segmente gemessen. Für beispielhafte Stegbreiten der Stege des mindestens einen endständigen Segments und der Stege des mindestens einen innenliegenden Segments wird auf die obigen Angaben verwiesen.

Der Grundkörper des erfindungsgemäßen Implantats kann vollständig oder teilweise aus einem biodegradierbaren Material bestehen. Derartige biodegradierbare Materialien sind metallische biodegradierbare Werkstoffe, insbesondere basierend auf Magnesium oder einer Magnesiumlegierung, beispielsweise WE43, Magnesium-Zink-Aluminium, Magnesium-Aluminium oder Magnesium-Zink-Kalzium. Hierbei werden bevorzugt höchstreine Magnesiumlegierungen verwendet, wie Magnesium-Zink-Aluminium mit 0-4 Gew-% Zn und 2-10 Gew-% A1 oder mit 1,5-7 Gew-% Zn und 0,5-3,5 Gew-% Al, wie Magnesium-Aluminium mit 5-10 Gew-% Al, insbesondere 5,5-7 Gew-%, besonders bevorzugt 6,25% Aluminium, wie Magnesium-Zink-Calcium mit 3-7 Gew-% Zn und 0,001-0,5 Gew-% Ca oder 0-3 Gew-% Zn und 0-0,6 Gew-% Ca. Derartige höchstreiche Magnesiumlegierungen weisen neben den genannten Legierungselementen weniger als 0,006 Gew-% andere Elemente (Verunreinigungen wie Fe, Cu, Co, Si etc. oder seltene Erden) auf. Die Verwendung von derartigen höchstreinen Magnesiumlegierungen für den Grundkörper des Implantats hat den Vorteil, dass sich die Bildung von Ausscheidungen sehr gut kontrollieren lässt. Durch die Kontrolle der Größe, des Volumens und der Zusammensetzung der Ausscheidungen lässt sich wiederum das Degradationsverhalten des Implantats sehr genau steuern. Bevorzugt wird das Degradationsverhalten des Implantats derart eingestellt, dass seine Stützfunktion für einen Zeitraum von mindestens 90 Tagen nach Implantation in das Gefäß erhalten bleibt und das Implantat nach einem Zeitraum von ca. einem Jahr nahezu degradiert, das heißt abgebaut ist.

Beispiele für geeignete biodegradierbare polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Das biodegradierbare Material kann auch teilweise aus einem metallischen biodegradierbaren Werkstoff und teilweise aus einer polymeren biodegradierbaren Verbindung bestehen.

In einer alternativen Ausgestaltung ist der Grundkörper des Implantats vollständig oder teilweise aus einem nicht biodegradierbaren Metall oder Metalllegierung wie Edelstahl, Titan, Cobalt-Chrom Legierung, Nickel-Titan Legierung, Platin oder ähnlichen geeigneten Metalllegierungen.

In einer weiteren bevorzugten Ausgestaltung weist das Implantat zumindest teilweise eine wirkstoffhaltige Polymerbeschichtung auf, wobei insbesondere der gesamte Grundkörper gleichmäßig beschichtet ist, d.h. kein Beschichtungsunterschied zwischen den Endsegmenten und den innenliegenden Segmenten besteht. Als Polymere können die im vorangehend genannten Polymere, insbesondere Polylactid (PLA) oder Poly-L-Lactid (PLLA) verwendet werden.

Als Wirkstoff können antiproliferative, antimigrative, antiangiogene, antiinflammatorische, antiphlogistische, zytostatische, zytotoxische und/oder antithrombotische Wirkstoffe, Antirestenose-Wirkstoffe, Corticoide, Sexualhormone, Statine, Epothilone, Porstacycline, Angiogeneseinduktoren verwendet werden. Insbesondere bevorzugt sind Paclitaxel und dessen Derivate und/oder Sirolimus und dessen Derivate.

Die Erfindung wird nachfolgend anhand eines Ausführungsbeispiels und unter Bezugnahme auf die Figuren erläutert. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbezügen.

Es zeigen schematisch:
- Fig. 1: ein Grundkörper eines erfindungsgemäßen Implantats im Zustand nach Elektropolitur, dargestellt in einer Abrollebene in einer Ansicht von oben,
- Fig. 2: separat den Ausschnitt D aus Fig. 1 in einer Vergrößerung,
- Fig. 3: separat den Ausschnitt C aus Fig. 1 in einer Vergrößerung,
- Fig. 4: separat den Ausschnitt B aus Fig. 1 in einer Vergrößerung und
- Fig. 5: ein erfindungsgemäßes Implantat im Zustand nach Elektropolitur in einer perspektivischen Ansicht von der Seite.

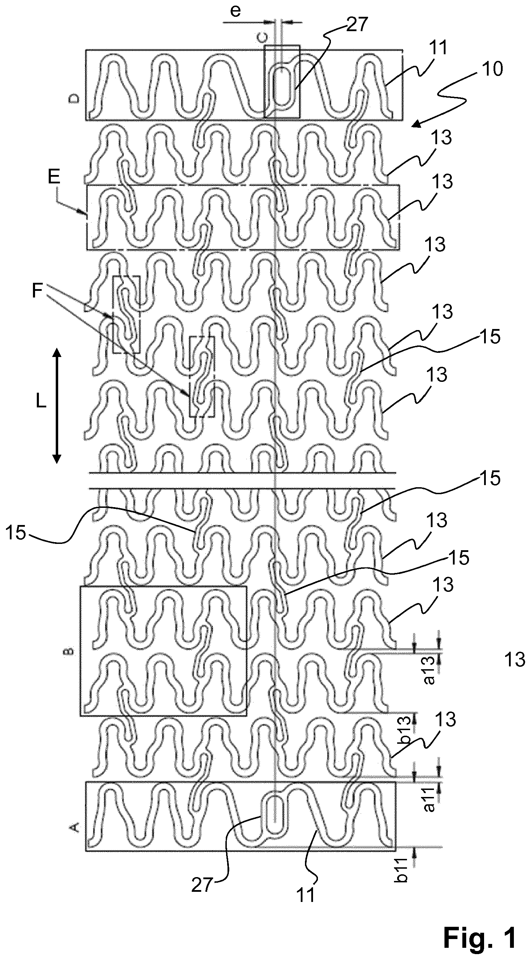

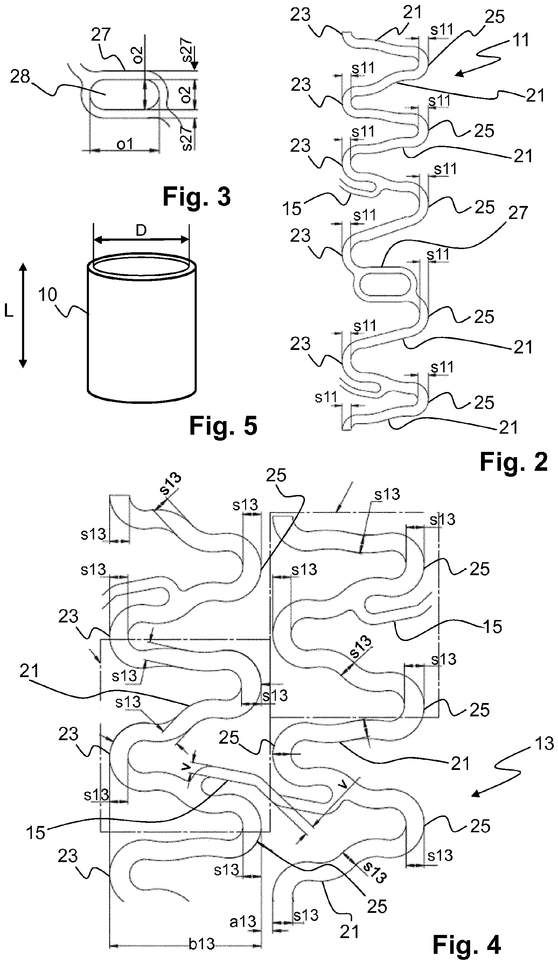

In Fig. 1 ist ein Grundkörper 10 eines Ausführungsbeispiels eines erfindungsgemäßen Implantats in Form eines Stents im Zustand nach Elektropolitur vor dem Komprimieren dargestellt, wobei zur besseren Veranschaulichung des Grundkörpers 10, der eine durchbrochene Struktur mit einer Vielzahl von in einzelnen Segmenten angeordneten Stegen aufweist, in eine Abrollebene abgerollt wurde. Fig. 5 veranschaulicht die Hohlzylinder-Form des Grundkörpers 10 im komprimierten Zustand, wenn dieser nicht abgerollt ist, ohne die Struktur des Grundkörpers 10 im Detail zu zeigen.

Im Zustand nach Elektropolitur weist der Grundkörper 10 einen Durchmesser auf, der dem expandierten Durchmesser nahe kommt, jedoch kleiner ist als der Zieldurchmesser im expandierten Zustand. Der Durchmesser des Grundkörpers entspricht hier im Wesentlichen dem Durchmesser des Rohres aus dem der Grundkörper hergestellt wurde.

Der Grundkörper 10 setzt sich aus einer Vielzahl von Segmenten 11, 13 zusammen, wobei in Fig. 1 insgesamt elf Segmente 11, 13 vollständig dargestellt sind. Die Segmente mit den Bezugszeichen 11 bilden endständige Segmente, da sie an den jeweiligen, in Längsrichtung L (Richtung der Längsachse des hohlzylindrischen Grundkörpers 10, siehe Fig. 5) angeordneten Enden des Grundkörpers 10 vorgesehen sind. Die übrigen Segmente 13 bilden innenliegende Segmente.

Jedes Segment 11, 13 ist mit dem jeweils benachbarten Segment 11, 13 mit jeweils zwei Verbindungsstegen 15 verbunden. Die Verbindungsstege 15 sind in den Ausschnitten F des Grundkörpers 10 in Fig. 1 hervorgehoben. Die Verbindungsstege 15 verlaufen bei dem dargestellten Ausführungsbeispiel geschwungen oder S-förmig, sie können aber in ihrem Mittelteil auch gerade verlaufen. Für die Verbindung benachbarter Segmente 11, 13 können in alternativen Ausführungsbeispielen auch nur ein einziger Verbindungssteg oder mehr als zwei Verbindungsstege vorgesehen sein.

Jedes Segment 11, 13 weist eine Vielzahl von Stegen auf, die sich im Wesentlichen in Längsrichtung L oder etwas schräg zu dieser erstrecken. Die Stege, die in Fig. 2 und 4 mit dem Bezugszeichen 21 versehen sind, können gerade oder gebogen oder geschwungen verlaufen, wobei verschiedene gerade und gebogene und geschwungene Stege in einem einzigen Segment angeordnet sein können. Jeweils zwei benachbarte Stege 21 werden durch Bögen verbunden, sodass eine mäanderförmige Struktur entsteht. Die Bögen bilden jeweils entweder ein Minima 23 bzw. ein Maxima 25 der mäanderförmigen Struktur (siehe Fig. 2 und 4).

In dem dargestellten Ausführungsbeispiel (siehe insbesondere Ausschnitte A, D und E der Fig. 1 sowie Fig. 2) weisen die innenliegenden Segmente 13 jeweils zwölf geschwungene Stege 21 sowie zwölf Bögen auf, wobei hiervon sechs Bögen Maxima 25 und sechs Bögen Minima 23 bilden. Die endständigen Segmente 11 setzen sich demgegenüber aus zehn Stegen 21 sowie zehn Bögen zusammen, wobei fünf Bögen Maxima 25 und fünf Bögen Minima 23 bilden. An einem Steg 21 des endständigen Segments 11 ist eine Öse 27 angeordnet, welche in Fig. 3 vergrößert dargestellt ist. Hierbei weist der erfindungsgemäße Stent sowohl im Bereich der innenliegenden Segmente 13 als auch im Bereich der endständigen Segmente 11 im Zustand nach Elektropolitur den gleichen oder im Wesentlichen den gleichen Außendurchmesser auf, der in Fig. 5 mit D bezeichnet ist. Im expandierten Zustand weisen die innenliegenden Segmente 13 den gleichen oder im Wesentlichen den gleichen Innendurchmesser wie die Endsegmente 11 auf. Leichte Unterschiede im Außendurchmesser D zwischen den endständigen Segmenten 11 und den innenliegenden Segmenten 13 können sich lediglich durch ein unterschiedliches Rückfederverhalten nach dem Komprimieren oder nach der Expansion aufgrund der unterschiedlichen mechanischen Charakteristika der innenliegenden Segmente 13 und der endständigen Segmente 11 ergeben. Im Wesentlichen ist der Außendurchmesser D für die innenliegenden Segmente 13 und die endständigen Segmente 11 gleich.

Andere Anzahlen von Minima, Maxima und Stegen in den innenliegenden Segmenten und in den endständigen Segmenten sind ebenfalls denkbar, wobei erfindungsgemäß die Anzahl der Minima des endständigen Segments um mindestens Eins kleiner ist als die Anzahl der Minima der innenliegenden Segmente.

Die in Fig. 3 im Detail dargestellte Öse 27 dient zur Anordnung eines Funktionselements bestehend aus radioopakem und/oder röntgenopakem Material in der inneren, durchgehenden Öffnung 28, um eine Sichtbarkeit des Stents beim Einbringen in den Körper zu gewährleisten. Durch die Reduktion der Minima in den endständigen Segmenten ist es möglich, das Volumen bzw. die sichtbare Fläche des Funktionselements zu erhöhen, sodass die Sichtbarkeit des Stents verbessert wird. Hierbei weist die Öse 27 und ihre innere Öffnung 28 in etwa eine elliptische Form auf, die eine, verglichen mit anderen Formen, große Fläche besitzt. Die Ellipse ist in dem Grundkörper 10 derart angeordnet, dass ihre Hauptachse parallel zur Längsrichtung L des Grundkörpers verläuft. Zudem sind die Hauptachsen der elliptischen Öffnungen 28 der beiden, an den beiden endständigen Segmenten 11 angeordneten Ösen 27 um eine Länge e in Umfangsrichtung (siehe Fig. 1) versetzt. Die Länge der Hauptachse o1 der Öffnung 28 der Öse 27 beträgt beispielsweise 800 µm, die Länge der Nebenachse o2 beispielsweise 350 µm. Die Stegbreiten s27 der Öse betragen z.B. 100 µm.

Die beiden endständigen Segmente 11 weisen eine Segmentbreite b11 von beispielsweise 1,36 mm auf, während die Segmentbreite b13 der innenliegenden Segmente 13 beispielsweise 1,25 mm beträgt (vergleiche Fig. 1). Andere Segmentbreiten sind ebenfalls denkbar, wobei vorzugsweise gelten soll: b11 > b13. Hierdurch wird die radiale Steifigkeit der endständigen Segmente 11 weiter reduziert.

In Fig. 1 sind ferner die Abstände zwischen den Segmenten eingezeichnet. Mit a11 wird der Abstand zwischen einem endständigen Segment 11 und einen benachbarten innenliegenden Segment 13 bezeichnet, während a13 der Abstand zwischen zwei innenliegenden Segmenten 13 ist. Um die axiale Steifigkeit im Übergangsbereich zu reduzieren, ist in dem dargestellten Ausführungsbeispiel eines Implantats a11 > a13, beispielsweise a11 = 115 µm und a13 = 95 µm.

Weiter wird ein besserer Übergang in der Steifigkeit von dem ungestenteten Gefäß zum gestenteten Gefäß erzielt, wenn die Stegbreiten s11 der endständigen Segmente 11 kleiner sind als die Stegbreiten s13 der innenliegenden Segmente, wobei die Segmente unterschiedliche Stegbreiten s11, s13 innerhalb eines Segments aufweisen können. Beispielsweise beträgt s11 zwischen 135 µm und 149 µm und s13 zwischen 150 µm und 165 µm. Gleiches gilt für unterschiedliche Stegdicken (nicht eingezeichnet) zwischen den Stegen der endständigen Segmente 11 und den Stegen der innenliegenden Segmente 13.

Weiter ist bei dem dargestellten Ausführungsbeispiel eines Implantats, um eine gute axiale Flexibilität sowie eine gute Biegeflexibilität des Stents bzw. des Grundkörpers 10 zu gewährleisten, die Stegbreite v der Verbindungsstege 15 kleiner als die Stegbreite s13 der Stege der innenliegenden Segmente 13 und die Stegbreite s11 der endständigen Segmente 11. Beispielsweise beträgt die Stegbreite v der Verbindungsstege 15 zwischen 80 µm und 90 µm.

Zudem weisen die Stege 21, Maxima 25 bzw. Minima 23 der endständigen Segmente 11 eine stärkere Verrundung der Kanten auf als die gleichen Elemente der mäanderförmigen Struktur der innenliegenden Segmente 13. Auch hierdurch wird die Störung der Strömung der Körperflüssigkeit beim Übergang zum Stent verringert.

Der erfindungsgemäße Stent kann in bekannter Weise mittels Laserschneiden aus einem röhrenförmigen Halbzeug und anschließendem Elektropolieren hergestellt werden. Danach wird ein wie die inneren Öffnung 28 der Öse geformtes Funktionselement aus röntgenopaken und/oder radioopaken Material in die Öffnung 28 eingesetzt und an der Öse 27, beispielsweise mittels Kleben, befestigt.

Zur Behandlung eines Patienten kann der erfindungsgemäße Stent beispielsweise auf den Ballon eines nicht dargestellten Katheters aufgecrimpt werden. Danach kann der Stent mittels des Katheters in den Körper des Patienten eingebracht und, beispielsweise entlang von Blutgefäßen, an die zu behandelnde Stelle vorgeschoben werden. Der Stent wird dann mittels des Ballons expandiert und hierdurch an der Wand des Gefäßes festgelegt. Der Stent dient dazu, das Gefäß offen zu halten und zu stützen. Der Katheter wird nach der Verankerung des Stents in dem Gefäß entfernt.

Der Grundkörper des Stents besteht aus einer höchstreinen Magnesium-AluminiumLegierung enthaltend 5,5 -7 Gew-% Aluminium (bevorzugt 6.25 Gew% Aluminium), maximal 0,006 Gew-% andere Elemente (Verunreinigungen wie Fe, Cu, Co, Si etc. oder seltene Erden) und Magnesium als restlichen Bestandteil. Der Stent weist ferner eine Sirolimus enthaltende PLLA Beschichtung auf, wobei die Beschichtung auf der luminalen Seite (Innenseite) des Stents dünner ist (2 - 7 µm) als auf der abluminalen Seite (Außenseite) des Stents (10 - 15 µm).

## Patentansprüche

1. Implantat mit einem durchbrochenen, hohlzylinderförmigen Grundkörper (10), der sich aus einer Vielzahl von durchbrochenen, hohlzylinderförmigen, in Längsrichtung (L) aneinandergereihten Segmenten (11, 13) zusammensetzt, die mit einander verbunden sind, wobei die Segmente jeweils ein endständiges Segment (11) an jedem in Längsrichtung angeordneten Ende des Grundkörpers (10) und mindestens ein innenliegendes Segment (13) umfassen, das in Längsrichtung (L) zwischen den zwei endständigen Segmenten (11) angeordnet ist, wobei jedes Segment (11, 13) eine Vielzahl von Stegen (21) aufweist, die zusammen eine in Umfangsrichtung umlaufende mäanderförmige Struktur mit Maxima (25) und Minima (23) ausbilden, wobei der Grundkörper (10) einen komprimierten Zustand und einen expandierten Zustand einnimmt, **dadurch gekennzeichnet, dass** die mäanderförmige Struktur mindestens eines endständigen Segments (11) eine geringere Anzahl von Minima (23) aufweist als das mindestens eine innenliegende Segment (13).

2. Implantat nach Anspruch 1, **dadurch gekennzeichnet, dass** im expandierten Zustand der Außendurchmesser des mindestens einen endständigen Segments dem Außendurchmesser des mindestens einen innenliegenden Segments entspricht.

3. Implantat nach einem der vorhergehenden Ansprüche, **dadurch gekennzeichnet** beide endständige Segmente (11) eine geringere Anzahl Minima (23) aufweisen als das mindestens eine innenliegende Segment (13).

4. Implantat nach einem der vorhergehenden Ansprüche, **dadurch gekennzeichnet, dass** an der mäanderförmigen Struktur mindestens eines endständigen Segments (11) des Grundkörpers (10) mindestens eine Öse (27) für die Anordnung eines Funktionselements bestehend aus einem röntgenopaken und/oder radioopaken Material vorgesehen ist, wobei die innere Öffnung (28) der Öse (27) vorzugsweise eine elliptische Form aufweist.

5. Implantat nach Anspruch 4, **dadurch gekennzeichnet, dass** jeweils ein Funktionselement bestehend aus einem röntgenopaken und/oder radioopaken Material in der mindestens einen Öse (27) angeordnet ist.

6. Implantat nach einem der vorhergehenden Ansprüche, **dadurch gekennzeichnet, dass** mindestens ein endständiges Segment (11) eine Segmentbreite (b11) aufweist, die größer ist als die Segmentbreite (b13) des mindestens einen innenliegenden Segments (13).

7. Implantat nach einem der vorhergehenden Ansprüche, **dadurch gekennzeichnet, dass** der Abstand (a11) eines endständigen Segments (11) von dem benachbarten innenliegenden Segment (13) größer ist als der Abstand (a13) zweier benachbarter innenliegender Segmente (13).

8. Implantat nach einem der vorhergehenden Ansprüche, **dadurch gekennzeichnet, dass** die Stege (21) mindestens eines endständigen Segments (11) eine geringere Breite (s11) und/oder Dicke und/oder eine stärkere Verrundung der Kanten aufweisen als die Stege (21) des mindestens einen innenliegenden Segments (13).

9. Implantat nach einem der vorhergehenden Ansprüche, **dadurch gekennzeichnet, dass** benachbarte Segmente (11, 13) mittels mindestens eines Verbindungsstegs (15) miteinander verbunden sind, wobei der mindestens eine Verbindungssteg (15) eine geringere Stegbreite (v) aufweist als die Stege (21) des mindestens einen innenliegenden Segments (13) und/oder die Stege des mindestens einen endständigen Segments.
